# EUROPEAN PATENT SPECIFICATION

(11) **EP 1 851 209 B1**
(45) Date of publication and mention of the grant of the patent: **30.05.2012**
(21) Application number: 06708091.1
(22) Date of filing: 08.02.2006
(51) Int. Cl.: C07D 285/01, C07D 401/12, C07D 403/12, A61K 31/425, A61K 31/427, A61P 25/28

(54) **[1,2,4]-DITHIAZOLI(DI)NE DERIVATIVES, INDUCERS OF GLUTHATHIONE-S-TRANSFERASE AND NADPH QUINONE OXIDO-REDUCTASE, FOR PROPHYLAXIS AND TREATMENT OF ADVERSE CONDITIONS ASSOCIATED WITH CYTOTOXICITY IN GENERAL AND APOPTOSIS IN PARTICULAR**
[1,2,4]-DITHIAZOLI(DI)N-DERIVATE, GLUTHATHION-S-TRANSFERASE-INDUKTOREN UND NADPH-CHINON-OXIDOREDUKTASE ZUR PROPHYLAXE UND BEHANDLUNG VON UNGÜNSTIGEN BEDINGUNGEN IN ZUSAMMENHANG MIT ZYTOTOXIZITÄT IM ALLGEMEINEN UND APOPTOSE IM BESONDEREN
DERIVES DE [1,2,4]-DITHIAZOLI(DI)NE, INDUCTEURS DE LA GLUTHATHIONE-S-TRANSFERASE ET DE LA NADPH QUINONE OXYDOREDUCTASE, DESTINES A LA PROPHYLAXIE ET AU TRAITEMENT D'ETATS NOCIFS ASSOCIES A LA CYTOTOXICITE EN GENERAL ET, PLUS PARTICULIEREMENT, A L'APOPTOSE

(30) Priority: 11.02.2005 US 651661 P; 11.02.2005 EP 05101004
(43) Date of publication of application: 07.11.2007
(73) Proprietor: Abbott Healthcare Products B.V., 1381 CP Weesp (NL)
(72) Inventor: FEENSTRA, Roelof W., 1381 CP Weesp (NL); KEIZER, Hiskias G., 1381 CP Weesp (NL); PRAS-RAVES, Maria L., 1381 CP Weesp (NL); VAN VLIET, Bernard J., 1381 CP Weesp (NL); VAN SCHARRENBURG, Gustaaf J., 1381 CP Weesp (NL)
(74) Representative: Verhage, Marinus
(86) International application number: PCT/EP2006/050744
(87) International publication number: WO 2006/084854

(56) References cited:
- GB-A- 1 351 412
- J. E. OLIVER: "The Reaction of Dithiazolium Cations with Sodium Azide" J. ORG. CHEM., vol. 4936, no. 22, 1971, pages 3465-3467, XP002384599
- J. E. OLIVER, A. B. DEMILO: "Imino-1,2,4-dithiazoles. I. Alkylation" J. ORG. CHEM., vol. 39, no. 15, 1974, pages 2225-2228, XP002384600
- H. W. LINDEN, J. GOERDELER: "Reaktion von Iminodithiazolen mit Sulfenen zu 5-gliedgrigen Sultamen" TETRAHEDRON LETT., no. 20, 1977, pages 1729-1732, XP002384601
- J. E. OLIVER: "Imino-1,2,4-dithiazoles. IV. Alkylation as a Probe of No-Bond Resonance" J. ORG. CHEM., vol. 39, no. 15, 1974, pages 2235-2239, XP002384602
- J. E. OLIVER, J. L. FLIPPEN: "Imino-1,2,4-dithiazoles. III. Thermal Decomposition of 5-(Dialkylamino)-3-substituted imino)-1,2,4-dithiazoles" J. ORG. CHEM., vol. 39, no. 15, 1974, pages 2233-2235, XP002384603
- J. E. OLIVER, R. T. BROWN: "Imino-1,2,4-dithiazoles. II. Dipolar Additions" J. ORG. CHEM., vol. 39, no. 15, 1974, pages 2228-2233, XP002384604
- G. L'ABBE, N. WEYNS: "5-Imino-1,2,4-thiadiazolidin-3-ones as masked 1,3-dipoles" BULL. SOC. CHIM. BELG., vol. 100, no. 2, 1991, pages 185-186, XP009067574
- G. L'ABBE ET AL.: "Isomerizations of 1,2,4-thiadiazolidines via thiapentalene intermediates" BULL. SOC. CHIM. BELG., vol. 99, no. 6, 1990, pages 391-392, XP009067601
- S. N. PANDEYA ET AL.: "Synthesis, anticonvulsant and analgesic activity of 1,2,4-dithiazoline derivatives" INDIAN JOURNAL OF PHARMACEUTICAL SCIENCE, vol. 44, no. 2, 1982, pages 31-33, XP009067579
- N. SIDDIQUI, S. N. PANDEYA: "Anticonvulsant and hypnotic activities of isodithiobiurets and 1,2,4-dithiazolines" INDIAN JOURNAL OF PHARMACOLOGY, vol. 24, no. 3, 1992, pages 171-173, XP009067580
- B. STELANDER ET AL.: "Synthesis and X-ray analysis of a new representative of the class of 3,4-diaza-trithiapentalenes" BULL. SOC. CHIM. BELG., vol. 86, no. 4, 1977, pages 291-298, XP009067626
- J. GOERDELER, J. ULMEN: "3.5-Bis-thiocarbamoylimino-1.2.4-dithiazo lidine: Verbindungen vom "no-bond resonance"-Typ?" CHEM. BER., vol. 105, 1972, pages 1568-1577, XP009067623
- J. GOERDELER ET AL.: "Zur Kenntnis der Cycloaddukte aus 3-Imino-3H-1,2,4-dithiazolen und Nitrilen: Herstellungsbedingungen und Molekülstruktur" CHEM. BER., vol. 118, 1985, pages 3241-3247, XP009067614
- N. G. ARGYROPOULOS: "Product class 2: 1,2,4-dioxazoles, 1,2,4-oxathiazoles and 1,2,4-dithiazoles" SCIENCE OF SYNTHESIS, vol. 13, 2004, pages 29-71, XP009067608
- I. IWATAKI, A. UEDA: "Studies of Dithiobiurets. II. The Thermal Decomposition of 1,1-Dimethyl-5-acetyl-2,4-dithiobiuret" BULL. CHEM. SOC. JPN., vol. 45, no. 10, 1972, pages 3220-3222, XP009067613
- I. IWATAKI: "Studies of Dithiobiurets. III. The Preparation and Properties of 3,5-Disubstituted 3H-1,2,4-Dithiazoles" BULL. CHEM. SOC. JPN., vol. 45, no. 12, 1972, pages 3572-3579, XP009067612
- J. GOERDELER ET AL.: "Reaktion von Heterocumulenen mit 5-Äthoxy-3-phenylimino-3H-1,2,4-dithiazol" CHEM. BER., vol. 109, 1976, pages 848-854, XP009067616
- J. GOERDELER ET AL.: "Reaktion von Iminen der Dithiazol- und Isothiazol-Reihe mit einigen einfachen Heterocumulenen" CHEM. BER., vol. 110, 1977, pages 285-294, XP009067618
- S. N. PANDEYA ET AL.: "Synthesis and Biological Activity of Isodithiobiurets, Dithiobiurets, and Dithiazoles" PHARMACEUTICAL RESEARCH, vol. 4, no. 4, 1987, pages 321-326, XP009067578
- J. E. OLIVER ET AL.: "5-(Dialkylamino)-1,2,4-dithiazole-3-thion es and 3,5-Disubstituted-1,2,4-dithiazolium Salts" J. HETEROCYCL. CHEM., vol. 9, 1972, pages 447-449, XP002384612
- PATENT ABSTRACTS OF JAPAN vol. 05, no. 1996, 31 May 1996 (1996-05-31) -& JP 08 027148 A (AGENCY OF IND. SCIENCE & TECHNOL.), 30 January 1996 (1996-01-30)
- PATENT ABSTRACTS OF JAPAN vol. 004, no. 129, 10 September 1980 (1980-09-10) -& JP 55 081804 A (NIPPON TOKUSHU NOYAKKU SEIZO KK), 20 June 1980 (1980-06-20)
- DATABASE BEILSTEIN [Online] Beilstein Institut zur Förderung der Wissenschaften; XP002390964 Database accession no. 6728135,6728136,6728988,6729911,6731373,67 32316
- C. P. JOSHUA, S. K. THOMAS: "SYNTHESIS OF 1,3,5-TRISUBSTITUTED 2,4-DITHIOBIURETS: THEIR CONVERSION INTO 4-ALKYL-3,5-DI(SUBSTITUTED IMINO)-1,2,4-DITHIAZOLIDINES AND TO RELATED BENZOTHIAZOLYLTHIOUREAS" AUST. J. CHEM., vol. 35, 1982, pages 405-409, XP002390975
- DATABASE CAPLUS [Online] CHEMICAL ABSTRACTS SERVICE, COLUMBUS, OHIO, US; 12 May 1984 (1984-05-12), XP002390963 retrieved from STN Database accession no. 1973:156910 & JP 48 028470 A (NIPPON SODA CO., LTD.) 14 April 1973 (1973-04-14)

## Description

The present invention relates to 5-imino-5H-[1,2,4]-dithiazol-3-yl-amine and [1,2;4]-dithiazolidine-3,5-diylidene-diamine derivatives as inducers of glutha-thione-S-transferase (GST) and NADPH quinone oxidoreductase (NQO), to methods for the preparation of these compounds and to novel intermediates useful for the synthesis of said [1,2,4]-dithiazoli(di)ne derivatives. The invention also relates to the use of a compound disclosed herein for the manufacture of a medicament giving a beneficial effect. A beneficial effect is disclosed herein or apparent to a person skilled, in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the invention specific com pounds disclosed herein are used for the manufacture of a medicament useful for prophylaxis and treatment of adverse conditions associated with cytotoxicity in general and apoptosis in particular.

Apoptosis is closely involved in morphogenesis and histogenesis in the development process, maintenance of homeostasis, and bio-defense, and it is cell death having an important role in maintaining individual lives. When the death process regulated by genes is congenitally or postnatally hindered, apoptosis is excessively induced or inhibited to cause functional disorders in various organs, and thus diseases. Drugs showing an apoptosis inhibitory activity can be used as agents for the prophylaxis and treatment of diseases which are thought to be mediated by promotion of apoptosis.

The goal of the present invention was to develop anti-apoptotic compounds with mechanisms of action different from any of those known in the art.

Surprisingly it was found that [1,2,4]-dithiazoli(di)ne derivatives are inducers of gluthathione-S-transferase (GST), NADPH quinone oxidoreductase (NQO), and other enzymes under control of the "antioxidant responsive element" (ARE). Upregulation of the activity of these enzymes may have beneficial effects in the treatment of neurodegenerative diseases and other diseases where free radical mediated cell degeneration and/or cell death plays a decisive role.

The invention relates to compounds of the general formula (I): 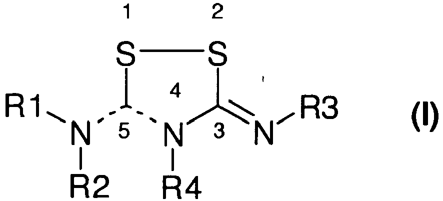 wherein
the dotted lines between carbon atom number 5 and its neighbouring nitrogen atoms represent single or double bonds, with the proviso that one of the two bonds is a single bond and one is a double bond, and the proviso that when the double bond is between carbon atom number 5 and nitrogen atom number 4, R4 does not exist and when the double bond is between carbon atom number 5 and the exo-nitrogen atom, R2 does not exist,
R1, R2, R3 and R4 independently represent a branched or unbranched alkyl(C₁₋₄) group, optionally containing sulphur atoms or sulphoxide, amide, ketone, thioketone or sulfone groups, and optionally substituted with halogen, cyano, mono- or dialkyl(C₁₋₄)amino, trifluoromethyl, (C₁₋₄)alkoxy or hydroxyl, or
R1 and R2 together with the nitrogen atom to which they are attached, form a 4 to 8 membered ring system in which other (substituted) hetero atoms chosen from N, O and S may be present, and which ring system may be optionally substituted with branched or unbranched alkyl(C₁₋₄), halogen, cyano, mono- or dialkyl(C₁₋₄)amino, trifluoromethyl, (C₁₋₄)alkoxy or hydroxyl or which ring system contains amide, ketone, thioketone, sulfone or sulfoxide functions,
R3 represents hydrogen, a branched or unbranched alkyl(C₁₋₄) group, optionally containing sulphur atoms or sulphoxide, amide, ketone, thioketone or sulfone groups, and optionally substituted with halogen, cyano, mono- or dialkyl(C₁₋₄)amino, trifluoromethyl, (C₁₋₄)alkoxy, S-alkyl(C₁₋₄), SH or hydroxyl, or
R3 represents an aryl group, optionally substituted with branched or unbranched alkyl(C₁₋₄), aryl, alkyl(C₁₋₄)aryl, SH, S-alkyl(C₁₋₄), halogen, cyano, mono- or dialkyl(C₁₋₄)amino, CF₃, OCF₃, SCF₃, nitro, hydroxy or (C₁₋₄)alkoxy groups, and tautomers, stereoisomers and N-oxides thereof, as well as pharmacologically acceptable salts, hydrates and solvates of said compounds of formula (I) and its tautomers, stereoisomers and N-oxides.

These compounds are new and are inducers of gluthathione-S-transferase (GST), NADPH quinone oxidoreductase (NQO).

The invention relates to racemates, mixtures of diastereomers as well as the individual stereoisomers of the compounds having formula (I). In the description of the substituents the abbreviation 'alkyl(C₁₋₄)' means 'methyl, ethyl, n-propyl, isopropyl, n-butyl, 2-butyl, isobutyl or 2-methyl-n-propyl'. 'aryl' means furyl, thienyl, pyrrolyl, oxazolyl, thiazolyl, imidazolyl, pyrazolyl, isoxazolyl, isothiazolyl, pyridyl, pyridazinyl, pyrimidinyl, pyrazinyl, 1,3,5-triazynyl, phenyl, indazolyl, indolyl, indolizinyl, isoindolyl, benzi[b]furanyl, benzo[b]thiophenyl, benzimidazolyl, benzthiazolyl, purinyl, quinolynyl, isochinolyl, chinolyl, phtalazinyl, quinazolinyl, quinoxalinyl, 1,8-naphthyridinyl, pteridinyl, naphthyl or azulenyl, preferably phenyl, pyridyl σ naphthyl. **'Optionally substituted'** means that a group may or may not be further substituted by one or more groups as indicated.

Prodrugs of the compounds mentioned above are in the scope of the present invention. Prodrugs are therapeutic agents which are inactive per se but are transformed into one or more active metabolites. Prodrugs are bioreversible derivatives of drug molecules used to overcome some barriers to the utility of the parent drug molecule. These barriers include, but are not limited to, solubility, permeability, stability, presystemic metabolism and targeting limitations (Medicinal Chemistry: Principles and Practice, 1994, ISBN 0-85186-494-5, Ed.: F. D. King, p. 215; J. Stella, "Prodrugs as therapeutics", Expert Opin. Ther. Patents, 14(3), 277-280, 2004; P. Ettmayer et al., "Lessons learned from marketed and investigational prodrugs", J.Med.Chem., 47, 2393-2404, 2004). Pro-drugs, i.e. compounds which when administered to humans by any known route, are metabolised to compounds having formula (1), belong to the invention. In particular this relates to compounds with primary or secondary amino or hydroxy groups. Such compounds can be reacted with organic acids to yield compounds having formula (1) wherein an additional group is present which is easily removed after administration, for instance, but not limited to amidine, enamine, a Mannich base, a hydroxyl-methylene derivative, an O-(acyloxymethylene carbamate) derivative, carbamate, ester, amide or enaminone.

N-oxides of the compounds mentioned above are in the scope of the present invention. Tertiary amines may or may not give rise to Noxide metabolites. The extend to what N-oxidation takes place varies from trace amounts to a near quantitative conversion. N-oxides may be more active than their corresponding tertiary amines or less active. Whilst N-oxides are easily reduced to their corresponding tertiary amines by chemical means, in the human body this happens to varying degrees. Some N-oxides undergo nearly quantitative reductive conversion to the corresponding tertiary amines, in other cases the conversion is a mere trace reaction or even completely absent. (M.H. Bickel: "The pharmacology and Biochemistry of N-oxides", Pharmaco-logical Reviews, 21(4), 325 - 355, 1969).

The invention particularly relates to compounds of the general formula (I^{a}): 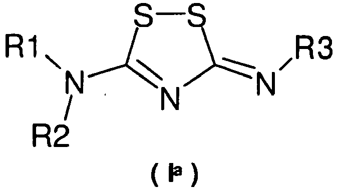 wherein
R1 and R2 independently represent a branched or unbranched alkyl(C₁₋₄) group, optionally containing sulphur atoms or sulphoxide, amide, ketone, thioketone or sulfone groups, and optionally substituted with halogen, cyano, mono- or dialkyl(C₁₋₄)amino, trifluoromethyl, (C₁₋₄)alkoxy or hydroxyl, or
R1 and R2 together with the nitrogen atom to which they are attached, form a 4 to 8 membered ring system in which other (substituted) hetero atoms chosen from N, O and S may be present, and which ring system may be optionally substituted with branched or unbranched alkyl(C₁₋₄), halogen, cyano, mono- or dialkyl(C₁₋₄)amino, trifluoromethyl, (C₁₋₄)alkoxy or hydroxyl or which ring system contains amide, ketone, thioketone, sulfone or sulfoxide functions,
R3 represents hydrogen, a branched or unbranched alkyl(C₁₋₄) group, optionally containing sulphur atoms or sulphoxide, amide, ketone, thioketone or sulfone groups, and optionally substituted with halogen, cyano, mono- or dialkyl(C₁₋₄)amino, trifluoromethyl, (C₁₋₄)alkoxy, S-alkyl(C₁₋₄), SH or hydroxyl, or
R3 represents an aryl group, optionally substituted with branched or unbranched alkyl(C₁₋₄), aryl, alkyl(C₁₋₄)aryl, SH, S-alkyl(C₁₋₄), halogen, cyano, mono- or dialkyl(C₁₋₄)amino, CF₃, OCF₃, SCF₃, nitro, hydroxy or (C₁₋₄)alkoxy groups.

More in particular the invention relates to compounds of the general formula (I^{a}) in which:
R1 and R2 independently represent branched or unbranched alkyl(C₁₋₄) group, or R1 and R2 together with the nitrogen atom to which they are attached, form a 5 to 6 membered ring system in which an oxygen atoms may be present, and which ring system may be optionally substituted with branched or unbranched alkyl(C₁₋₄), R3 represents an aryl group, optionally substituted with branched or unbranched alkyl(C₁₋₄), aryl, alkyl(C₁₋₄)aryl, SH, S-alkyl(C₁₋₄), halogen, cyano, mono- or dialkyl(C₁₋₄)amino, CF₃, OCF₃, SCF₃, nitro, hydroxy or (C₁-₄)alkoxy groups.

Even more particular the invention relates to compounds of the general formula (I^{a}) in which:
R1 and R2 independently represent a methyl group, or R1 and R2 together with the nitrogen atom to which they are attached, form a 5 to 6 membered ring system in which one oxygen atom may be present, and which ring system may be optionally substituted with a methyl group, R3 represents a phenyl or pyridyl group, optionally substituted with branched or unbranched alkyl(C₁₋₄), benzyl, SH, SCH₃, halogen, cyano, CF₃, OCF₃, SCF₃, nitro, hydroxy or methoxy groups.

Also in particular the invention relates to compounds of the general formula (I^{b}) 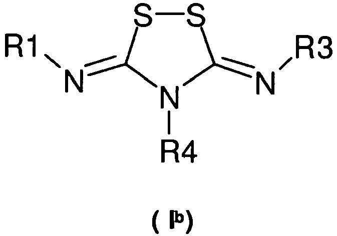 wherein
R1, R3 and R4 independently represent a branched or unbranched alkyl(C₁₋₄) group, optionally containing sulphur atoms or sulphoxide, amide, ketone, thioketone or sulfone groups, and optionally substituted with halogen, cyano, mono- or dialkyl(C₁₋₄)amino, trifluoromethyl, (C₁₋₄)alkoxy or hydroxyl, or
an aryl group, optionally substituted with branched or unbranched alkyl(C₁₋₄), aryl, alkyl(C₁₋₄)aryl, SH, S-alkyl(C₁₋₄), halogen, cyano, mono- or dialkyl(C₁₋₄)- amino, CF₃, OCF₃, SCF₃, nitro, hydroxy or (C₁₋₄)alkoxy groups,

And more in particular the invention relates to compounds of the general formula (I^{b}) in which:
R1, R3 and R4 independently represent branched or unbranched alkyl(C₁₋₄) group, or an aryl group, optionally substituted with branched or unbranched alkyl(C₁₋₄), aryl, alkyl(C₁₋₄)aryl, halogen, CF₃, OCF₃, SCF₃, nitro, hydroxy or methoxy groups,

Even more particular the invention relates to compounds of the general formula (I^{b}) in which:
R1, R3 and R4 independently represent a methyl group or a phenyl group, optionally substituted with branched or unbranched alkyl(C₁₋₄), halogen, CF₃, OCF₃, hydroxy or methoxy groups.

### GENERAL ASPECTS OF SYNTHESES

Compounds with the general formulae (I^{a}) or (I^{b}) can be obtained according to three methods (schemes A.1, B.1 and C.1):

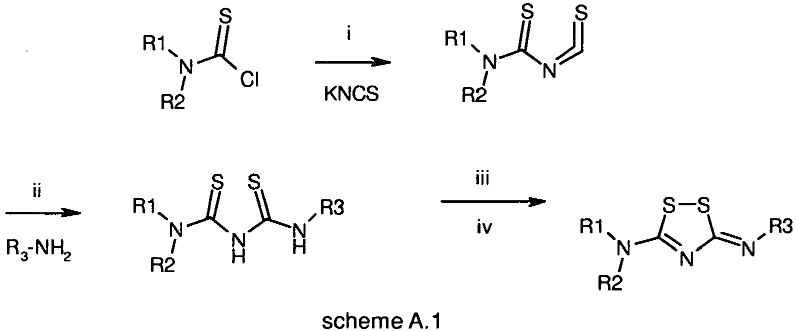

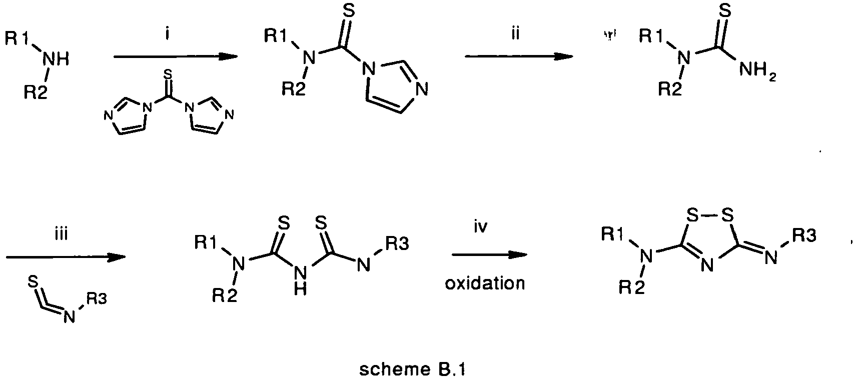

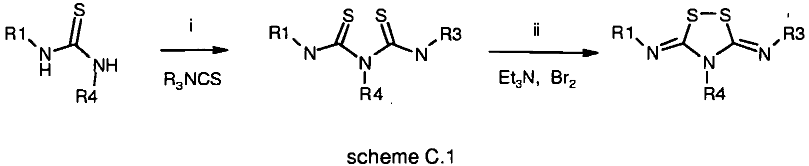

The selection of the particular synthetic procedures depends on factors known to those skilled in the art such as the compatibility of functional groups with the reagents used, the possibility to use protecting groups, catalysts, activating and coupling reagents and the ultimate structural features present in the final compound being prepared.

Pharmaceutically acceptable salts may be obtained using standard procedures well known in the art, for example by mixing a compound of the present invention with a suitable acid, for instance an inorganic acid such as hydrochloric acid, or with an organic acid.

The compounds of the invention of the general formula (I), as well as the salts thereof, are inducers of gluthathione-S-transferase (GST), NADPH quinone oxidoreductase (NQO), and other enzymes under control of the "antioxidant responsive element" (ARE). Upregulation of the activity of these enzymes may have beneficial effects in the treatment of neurodegenerative diseases and other diseases where free radical mediated cell degeneration and/or cell death (apoptosis) plays a decisive role. The compounds of the invention are active at doses in the range of 0.1 - 100 mg/kg after oral administration, and are useful in prophylaxis and treatment of adverse conditions associated with apoptosis. Said conditions are for instance: **neurodegenerative disorders** such as e.g. ischemic stroke, traumatic brain injury, acute disseminated encephalomyelitis, amyotrophic lateral sclerosis (ALS), retinitis pigmentosa, mild cognitive impairment, Alzheimer's disease, Pick's disease, senile dementia, progressive supranuclear palsy, subcortical dementias, Wilson disease, multiple infarct disease, arteriosclerotic dementia, AIDS associated dementia, cerebellar degeneration, spinocerebellar degeneration syndromes, Friedreichs ataxia, ataxia telangiectasia, epilepsy related brain damage, spinal cord injury, restless legs syndrome, Huntington's disease and Parkinson's disease, striatonigral degeneration, cerebral vasculitis, mitochondrial encephalo-myopathies, neuronal ceroid lipofuscinosis, spinal muscular atrophies, lysosomal storage disorders with central nervous system involvement, leukodystrophies, urea cycle defect disorders, hepatic encephalopathies, renal encephalopathies, metabolic encephalopathies, porphyria, bacterial or viral meningitis and meningoencephalitis, prion diseases, poisonings with neurotoxic compounds, Guillain Barre syndrome, chronic inflammatory neuropathies, polymyositis, dermatomyositis, radiation-induced brain damage; irritable bowel disease and inflammatory bowel diseases, Crohn's disease and ulcerative colitis, coeliac disease, Helicobacter pylori gastritis and other infectious gastritides, necrotizing enterocolitis, pseudomembranous enterocolitis, radiation-induced enterocolitis, lymphocytic gastritis, graft-versus-host disease, acute and chronic pancreatitis; **hepatic diseases** such as e.g. alcoholic hepatitis, viral hepatitis, metabolic hepatitis, autoimmune hepatitis, radiation-induced hepatitis, liver cirrhosis, hemolytic uremic syndrome, glomerulonephritis, lupus nephritis, **viral diseases** such fulminant hepatitis: **joint-diseases** such as trauma and osteoarthritis; **immuno-suppression or immunodeficiency,** in particular autoimmune diseases like idiopathic inflammatory myopathy, chronic neutropenia, thrombotic thrombocytopenic purpura, rheumatoid arthritis, idiopathic thrombocytopenic purpura, autoimmune haemolytic syndromes, antiphospholipid antibody syndromes, myocarditis, multiple sclerosis and its diagnostic sub-classifications relapsing-remitting multiple sclerosis, secondary progressive multiple sclerosis, primary progressive multiple sclerosis, progressive relapsing multiple sclerosis, acute multiple sclerosis, benign relapsing multiple sclerosis or asymptomatic multiple sclerosis, neuromyelitis optica (Devic's syndrome), lymphocytic hypophysitis, Grave's disease, Addison's disease, hypoparathyroidism, type 1 diabetes, systemic lupus erythematodes, pemphigus vulgaris, bullous pemphigoid, psoriatic arthritis, endometriosis, autoimmune orchitis, autoimmune erectile dysfunction, sarcoidosis, Wegener's granulomatosis, autoimmune deafness, Sjögren's disease, autoimmune uveoretinitis, interstitial cystitis, Goodpasture's syndrome and fibromyalgia; **myelodysplasias** such as aplastic anemia; **dermatological diseases** including pemphigous vulgaris, dermatomyositis, atopic dermatitis, Henoch-Schonlein purpura, acne, systemic sclerosis, seborrhoeic keratosis, cutaneous mastocytosis, chronic proliferative dermatitis, dyskeratosis, scleroderma, interstitial granulomatous dermatitis, psoriasis, bacterial infections of the skin, dermatomycoses, lepra, cutaneous leishmaniasis, vitiligo, toxic epidermal necrolysis, Steven Johnson syndrome, sebaceous adenoma, alopecia, photodamage of the skin, lichen sclerosus, acute cutaneous wounds, incontinentia pigmenti, thermal damage of the skin, exanthematous pustulosis, lichenoid dermatosis, cutaneous allergic vasculitis, cytotoxic dermatitis; **diseases of the inner ear** such as e.g. acoustic trauma-induced auditory hair cell death and hearing loss, aminoglycoside induced auditory hair cell death and hearing loss, ototoxic drug-induced hearing loss, perilymphatic fistula, cholesteatoma, cochlear or vestibular ischemia, Meniere's disease, radiation-induced hearing loss, hearing loss induced by bacterial or viral infections and idiopathic hearing loss; **transplantation:** graft-versus-host disease, acute and chronic rejection of heart-, lung-, kidney-, skin- corneal-, bone marrow- or liver-transplants; **wound healing** and **tissue rejection.**

### PHARMACEUTICAL PREPARATIONS

The compounds of the invention can be brought into forms suitable for administration by means of usual processes using auxiliary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxillary substances which may be mentioned are magnesium carbonate, titanium dioxide, lactose, mannitol and other sugars or sugar alcohols, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art. In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### PHARMACOLOGICAL METHODS

### Spectrophotometric determination of EC 2.5.1.18 (GST) induction

Rat PC-12 cells are incubated for 24 hours with a test compound. GST activity is measured using 1-chloro-2,4-dinitrobenzene (CDNB) and gluthathine (GSH) as substrates. The enzyme catalyses the reaction converting these substrates into S-(2,4-dinitrophenyl)glutathione, which product is measured spectrophoto-metrically at 340 nm. The effect of the test compound is expressed as pEC₅₀: the negative logarithm of the molar concentration of the compound which causes 50% induction of the enzyme activity. Also the maximal percentage enzyme induction is determined. (see Drukarch et al. "Anethole dithiolethione prevents oxidative damage in gluthathione-depleted astrocytes", Eur. J. Pharmacol., 329, 259-262, 1997).

### Spectrophotometric determination of EC 1.6.99.2 (NQO) induction

Rat PC-12 cells are incubated for 24 hours with a test compound. NAD(P)H: oxidoreductase (NQO, EC 1.6.99.2) activity is measured using menadione as substrate and MTT as readout compound. The formation of formazan from MTT is measured spectrophotometrically at 540 nm. The percentage stimulation (PS) relative to the maximal effect of the reference com pound D3T is calculated. The maximal percentage stimulation with the concentration which induced the maximal percentage as condition and the pEA₅₀ value (the negative logarithm of the molar concentration of the compound which caused the same half maximal induction of the enzyme activity as the reference compound D3T) are recorded (*see* Murphy et al., "Enhanced NAD(P)H:Quinone reductase activty defences in neuronal cells prevents free radical-mediated damage" J. Neurochem., 71 69-77, 1998 and Prochaska et al., Analytical Biochemistry, 169, 328-336 1988).

### DOSE

The potency of the compounds of the invention as inducers of gluthathione-S-transferase (GST), NADPH quinone oxidoreductase (NQO), was determined as described above. From the potency measured for a given compound of formula (I), one can estimate a theoretical lowest effective dose. At a concentration of the compound equal to twice the measured EC₅₀ the enzyme activity is likely to be doubled. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 -1000 mg/kg, preferably 0.1-100 mg/kg of patient's bodyweight.

### TREATMENT

The term "treatment" as used herein refers to any treatment of a mammalian, preferably human condition or disease, and includes: (1) preventing the disease or condition from occurring in a subject which may be predisposed to the disease but has not yet been diagnosed as having it, (2) inhibiting the disease or condition, i.e., arresting its development, (3) relieving the disease or condition, i.e., causing regression of the condition, or (4) relieving the conditions caused by the disease, i.e., stopping the symptoms of the disease.

### EXAMPLES

### EXAMPLE 1: MATERIALS AND METHODS

All reactions involving moisture sensitive compounds were carried out under a dry nitrogen atmosphere. All other commercially available chemicals were used without further purification. Reactions were monitored by using thin-layer chromatography (TLC) on silica coated plastic sheets (Merck silica gel 60 F254) with the indicated eluent. The compounds were visualised by UV light (254 nm) or I₂. Flash chromatography refers to purification using the indicated eluent and Acros silica gel (0.030-0.075 mm). Nuclear magnetic resonance spectra (¹H NMR) were determined in the indicated solvent. Coupling constants *J* are given in Hz. Peakshapes in the NMR spectra are indicated with the symbols 'q' (quartet), 'dq' (double quartet), 't' (triplet), 'dt' (double triplet), 'd' (doublet), 'dd' (double doublet), 's' (singlet), 'bs' (broad singlet) and 'm' (multiplet).

### EXAMPLE 2: SYNTHESES OF SPECIFIC COMPOUNDS

### SYNTHESIS ROUTE A:

The used thiocarbamoylchlorides are either commercially available or accessible via methods described in Gördeler et al., Chem.Ber., 103(1970)3393 and von Braun et al., Berichte Deutsche Chem.Ges., 36(1903)2274

### Compound A2:

### Procedure A (scheme A.1) steps i + ii:

12.3 grams (99 mmol) of N,N-dimethylthiocarbamoyl chloride were dissolved in 60 ml of acetonitrile. While stirring, a solution of 9.9 grams (99 mmol) of potassium isothiocyanate (KSCN) in 180 ml of acetonitrile, was added to the reaction mixture. Then the mixture was heated to reflux temperature, after which refluxing was continued for three minutes. Subsequently the reaction mixture was added to a stirred solution of 13.5 grams (90 mmol) 4-n-butyl-aniline in 100 ml of acetonitrile. The latter mixture was stirred for a short time after which the solvent was removed *in vacuo.* Water was added on which the residueal oil turned into a solid. The solid/water mixture was made into a suspension after which it was filtered. The residuee was washed with water, subsequently with a little isopropanol after which it was allowed to dry. Yield of crude product: 24.4 gram (92%). Recrystallization from isopropanol (ca. 200 ml) yielded 16.7 grams (63%) of the corres ponding dithiobiuret, m.p.:89.5-91 °C. ¹H NMR (90 MHz, d⁶-DMSO) δ (ppm): 0.93 (t, 3H), 1.15-2.05 (m, 4H), 2.61 (t, 3H), 3.35 (s, 6H), 7.18 (d, 2H), 7.55 (d, 2H), 9.79 (s, 2H).

### Procedure A1 (scheme A1) steps iii + iv:

11.8 grams (40 mmol) of the previously prepared dithiobiuret was solved in 300 ml of alcohol. While stirring, a solution containing 13.5 grams of iodine (53.2 mmol) and 16.7 potassium iodide (100 mmol) in 20 ml of water was added until the iodine color did not disappear anymore. The solution was allowed to stand upon which crystals formed. The mixture was filtered, the residue washed with alcohol, isopropanol and petroleum ether, yielding 13.4 grams (80%) of the mono hydrogen iodide salt. *Step iv:* 10.5 grams of the latter was suspended in water (250 ml) and, while stirring, 14 ml of 2N KOH was added. After 1.5 hours the suspension was filtered, the residue washed with water, a little isopropanol and petroleum ether, yielding 6.5 grams. The latter amount was recrystallized from 100 ml of ligroin, yielding 3.3 grams (45%) of pure product **A2** as a free base. M.p.: 81-82.5 °C. ¹H NMR (90 MHz, CDCl₃) δ (ppm): 0.93 (t, 3H), 1.1-1.9 (m, 4H), 2.60 (t, 2H), 3.35 (s, 6H), 6.94 and 7.16 (dd, 4H).

### Compound A19:

### Procedure A (scheme A.1) steps i to iv:

2 grams (16.3 mmol) of dimethylthiocarbamoylchloride and 1.58 grams of KNCS ( 16.3 mmol) were suspended in acetonitrile and heated to reflux for 2 minutes using a hotgun. To the resulting hot mixture was added 1.97 grams (16.3 mmol) of (2,6)-dimethylaniline in 10 ml of acetonitrile. After 1 hour the mixture was filtered and the filtrate was concentrated. The resulting oil containing the dithiobiuret was dissolved in 40 ml of ethanol and, a solution of 6.7 grams of iodine and 8.4 grams of potassium iodide in 20 ml of water, was added until the solution did not change color. Stirring was continued for 1 hour after which the reaction mixture was filtered, the residue washed with water, 2M KOH and again water. Subsequently the residue was washed with diethyl ether and allowed to dry, yielding 2.2 grams (49%) of the free base **A19** as a yellowish solid. M.p.: 236.3-239.2 °C (DSC). ¹H-NMR (200 MHz, d⁶-DMSO) δ (ppm): 2.28 (s, 6H), 2.57 (s, 6H), 7.16-7.45 (m, 4H). Mass (Cl): (M⁺+1): 266.

According to the syntheses given above, the compounds **A1-A22** (table A), all with the general formula (1^{a}), were prepared:

| **compound** | **R1** | **R2** | **R3** | **melting point °C** |
|---|---|---|---|---|
| | | | | |
| **A1** | Me | Me | Ph | 216 (decomp) |
| **A2** | Me | Me | 4-(*n*-But)-Ph | 81-82.5 |
| **A3** | Me | Me | 4-MeO-Ph | 129.5-131.5 |
| **A4** | Me | Me | 3-Cl-Ph | 113-114.5 |
| **A5** | Me | Me | 2,6-(di-Cl)-Ph | 191.5-193.5 |
| **A6** | Me | Me | 3-NO₂-Ph | 134.5- 136.5 |
| **A7** | -(CH₂)₄- | | Ph | 149-151.5 |
| **A8** | -(CH₂)₂O(CH₂)₂- | | Ph | 175.5-176.5 |
| **A9** | Me | Me | 2-Me-Ph | 138.5-140 |
| **A10** | Me | Me | 4-NO₂-Ph | 170-171.5 |
| **A11** | Me | Me | 4-F-Ph | 137-138.5 |
| **A12** | Me | Me | 2-(*i*-Pr)-Ph | 194.6-197.5 |
| **A13** | Me | Me | 2-F-Ph | 126.8-128.2 |
| **A14** | Me | Me | 2-MeS-Ph | 119.4-120.3 |
| **A15** | Me | Me | 3-Me-Ph | 201-204 |
| **A16** | Me | Me | 2-Et-Ph | 177.8-184 |
| **A17** | Me | Me | 2-MeO-Ph | 148.5-152 |
| **A18** | Me | Me | 2-CF₃-Ph | 129.1-130.8 |
| **A19** | Me | Me | 2,6-(di- Me)-Ph | 236.3-239.2 |
| **A20** | Me | Me | 2-pyridyl | 166-173 |
| **A21** | Me | Me | 2-benzyl-Ph | 176-182 |
| **A22** | Me | Me | 3-pyridyl | 83-90 |

| | | | | |
|---|---|---|---|---|
| (*in all cases the compounds were prepared as free bases, except for A1 which was prepared as the monohydrogen iodide. Abbreviations used are: Me = methyl; Ph = phenyl; Et = ethyl; But = butyl; i-Pr = iso-propyl*;) | | | | |

### SYNTHESIS ROUTE B:

### Compound B4:

### Procedure B (scheme B.1) step i + ii:

10 grams (56 mmol) of thiocarbonyldiimidazole was dissolved in THF (400 ml). 4.78 grams (56 mmol) of piperidine was added and stirred at 50°C for 3 hours. The mixture was partially concentrated and subsequently mixed with 500 ml 7N NH₃ in methanol, after which it was put away in a sealed vessel overnight at 45 °C. At room temperature the reaction mixture was partially concentrated after which it was cooled. The precipitate containing the thiourea derivative was collected and dried to give 1.3 g (16%) as a grey solid.

### Procedure B (scheme B.1) steps iii + iv:

1 gram (6.85 mmol) of the urea derivative of the previous step was dissolved in 40 ml of acetonitrile, after which 500 mg of powdered KOH was added and the mixture was stirred for 30 minutes at room temperature. Subsequently 1.02 grams (6.85 mmol) of 2-methylphenylisothiocyanate was added and the reaction mixture was stirred for 2 hours. The mixture was filtered and the filtrate concentrated. The resulting oil was dissolved in ethanol (40 ml) and a solution, of 6.7 grams of iodine and 8.4 grams of potassium iodide in 20 ml of water, was added until the solution did not change color. Stirring was continued for 1 hour after which the reaction mixture was filtered, the residue washed with water, 2M KOH and again water. Subsequently the residue was washed with diethyl ether and allowed to dry, yielding 781 milligrams (39%) of the free base **B4** as an off white solid. M.p.: 104.3-112.2 °C (DSC). ¹H-NMR (200 MHz, d⁶-DMSO) δ (ppm): 1.77 (s, 6H), 2.31 (s, 3H), 3.78 (broad, 4H), 6.95-7.40 (m, 4H). Mass (Cl): (M⁺+1): 292.

According to the synthesis given above, the compounds **B1-B16** (table B), all with the general formula (1 a) were prepared.

| **cmp** | **R1 and R2** | **R3** | **melting point °C** |
|---|---|---|---|
| **B1** | -(CH₂)₂O(CH₂)₂- | 2-Me-Ph | 139-142 |
| **B2** | -(CH₂)₄- | 2-Me-Ph | 126.8-128 |
| **B3** | -(CH₂)₂NMe(CH₂)₂- | 2-Me-Ph | 134-142 |
| **B4** | -(CH₂)₅- | 2-Me-Ph | 104.3-112.2 |
| **B5** | -(CH₂)₂O(CH₂)₂- | 3-Me-Ph | 217-226 |
| **B6** | -(CH₂)₂O(CH₂)₂- | 2-MeO-Ph | 160-164 |
| **B7** | -(CH₂)₂O(CH₂)₂- | 2-F-Ph | 144-149 |
| **B8** | -(CH₂)₂O(CH₂)₂- | 2-CF₃-Ph | 138-140.5 |
| **B9** | -(CH₂)₂O(CH₂)₂- | 2-MeS-Ph | 160-168 |
| **B10** | -(CH₂)₂O(CH₂)₂- | 2-Et-Ph | 195.6 |
| **B11** | -(CH₂)₂S(CH₂)₂- | 2-Et-Ph | 145.2 |
| **B12** | -(CH₂)₂S(CH₂)₂- | 2-F-Ph | 178.7 |
| **B13** | -CH₂-CH(CH₃)OCH(CH₃)-CH₂- | 2-Et-Ph | 182.2-188.1 |
| **B14** | -CH₂-CH(CH₃)OCH(CH₃)-CH₂- | 2-F-Ph | 199.4-203.9 |
| **B15** | -CH₂-CH(CH₃)OCH(CH₃)-CH₂- | 2-MeO-Ph | 193.4-196.5 |
| **B16** | -(CH₂)₂O(CH₂)₂- | 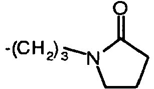 | 179.5-183 |

| | | | |
|---|---|---|---|
| *(in all cases the compounds were prepared as free bases)* | | | |

### SYNTHESIS ROUTE C:

### Compound C10:

### Procedure C (scheme C.1) step i:

While stirring, 3.4 grams (32.6 mmol) was dissolved in 100 ml of acetonitile after which 1.3 grams (32.6 mmol) of powdered NaOH were added. Stirring wars continued for 3 hours. Then 5 grams (32.6 mmol) of 4fluorophenylisothiocyanate were added after which the mixture was allowed to react overnight. Water and *tert-*butyl methyl ether (TBME) were added as well as 5 ml of concentrated hydrochloric acid. After shaking the biphasic mixture, the organic layer was separated and concentrated in vacuo. The residu was taken up in toluene and filtered. An oily substance separated and was removed from the filtrate. The filtrate was concentrated in vacuo, the residu taken up in TBME and 1M NaOH. The aqueous layer was washed with TBME three times. Subsequently, the aqueous layer was acidified with concentrated HCl after which extraction took place with TBME (3x). The combined organic fractions were concentrated in vacuo to yield 2.5 grams (30%) of the dithiobiuret derivative as a yellow solid.

### Procedure C1 (scheme C1) step ii:

2.5 grams (9.7 mmol) of the dithiobiuret prepared in step i, were dissolved in 80 ml of dichloromethane to which a solution of 0.5 ml (9.8 mmol, d=3.119 g/ml) of bromine in 20 ml of dichloromethane, was added dropwise. The reaction mixture was stirred for 30 minutes and the formed precipitate was filtered. The residu was washed with dichloromethane and TBME and then allowed to dry in vacuo which yielded 1.8 gram of an orange solid. The latter amount was suspended in dichloromethane and treated with 4.21 ml (30 mmol, 3 eq, d=0.72 g/ml) of triethylamine, the whole being stirred for 30 minutes. The reaction mixture was washed with water (3x), after which the organic layer was concentrated in vacuo. The residu was recrystallized twice from ethanol, yielding 275 mg (11%) of yellow product **C10**. M.p.: 81.2-84.4 °C (DSC). ¹H-NMR (200 MHz, CDCl₃) δ (ppm): 3.26 (s, 3H), 3.57 (s, 3H), 6.95-7.20 (m, 4H).

According to the syntheses given above, the compounds **C1-C11,** all with the general formula (I^{b}), (table A) were prepared.

| **compound** | **R1** | **R4** | **R3** | **melting point °C** |
|---|---|---|---|---|
| | | | | |
| **C1** | Me | Me | Me | 100-102 |
| **C2** | Me | Me | Ph | 80-82 |
| **C3** | Me | Me | 2-Me-Ph | 100-101.4 |
| **C4** | Me | Me | 4-Me-Ph | 113.2-114.5 |
| **C5** | Me | Me | 2-(*i*-Pr)-Ph | 80.5-82 and 293-296 |
| **C6** | Me | Me | 2-MeO-Ph | 85.7-88.6 and 291-296 |
| **C7** | Me | Me | 4-MeO-Ph | 54.6-56.2 and 286-295 |
| **C8** | Me | Me | 2-Cl-Ph | 92.5-95.3 |
| **C9** | Me | Me | 2,6-(di-Cl)-Ph | 82.8-83.8 |
| **C10** | Me | Me | 4-F-Ph | 81.2-84.4 |
| **C11** | Me | Me | 3-F-Ph | 76.5-77.6 |

### (in all cases the compounds were prepared as free bases)

The specific compounds of which the synthesis is described above are intended to further illustrate the invention in more detail, and therefore are not deemed to restrict the scope of the invention in any way. Other embodiments of the invention will be apparent to those skilled in the art from consideration of the specification and practice of the invention disclosed herein. It is thus intended that the specification and examples be considered as exemplary only, with a true scope and spirit of the invention being indicated by the claims.

### EXAMPLE 3: FORMULATION OF COMPOUND A10

**For oral (*p.o*.) administration:** to the desired quantity (0.5-5 mg) of the solid compound A10 in a glass tube, some glass beads were added and the solid was milled by vortexing for 2 minutes. After addition of 1 ml of a solution of 1% methylcellulose in water and 2% (v/v) of Poloxamer 188 (Lutrol F68), the compound was suspended by vortexing for 10 minutes. The pH was adjusted to 7 with a few drops of aqueous NaOH (0.1 N). Remaining particles in the suspension were further suspended by using an ultrasonic bath.

**For intraperitoneal (*i.p*.) administration:** to the desired quantity (0.5-15 mg) of the solid compound A10 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE 4: PHARMACOLOGICAL TESTRESULTS

Some enzyme induction data obtained according to the protocols given above are shown in the table below.

| | **ENZYME INDUCTION** | |
|---|---|---|
| | | |

| | **GST** | **NQO** |
|---|---|---|
| | | |

| **Compound** | **pEC₅₀** | **pEA₅₀** |
|---|---|---|
| | | |
| **A2** | **5.2** | **5.2** |
| **A10** | **5.4** | **5.8** |
| **B5** | **5.3** | **5.7** |
| **C2** | **5.8** | **6.2** |
| **C3** | **5.8** | **5.7** |
| **C11** | **5.6** | **5.3** |

## Claims

1. Compounds of the general formula (I): 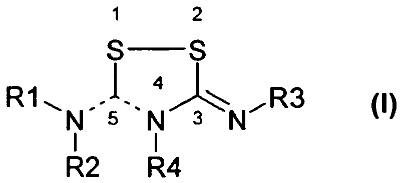 wherein the dotted lines represent single or double bonds, selected from the group consisting of compounds with the substituents:
| **R1** | **R2** | **R3** | **R4** |
|---|---|---|---|
| | | | |
| CH₃ | CH₃ | 2-pyridyl | - |
| CH₃ | CH₃ | 3-pyridyl | - |
| CH₃ | CH₃ | 2-F-phenyl | - |
| CH₃ | CH₃ | 2-CH₃-phenyl | - |
| CH₃ | CH₃ | 2-CF₃-phenyl | - |
| CH₃ | CH₃ | 2-CH₃CH₂-phenyl | - |
| CH₃ | CH₃ | 2-(*i*-Pr)-phenyl | - |
| CH₃ | CH₃ | 2-CH₃O-phenyl | - |
| CH₃ | CH₃ | 2-CH₃S-phenyl | - |
| CH₃ | CH₃ | 2-benzyl-phenyl | - |
| CH₃ | CH₃ | 2,6-Cl₂-phenyl | - |
| CH₃ | CH₃ | 2,6-(CH₃)₂-phenyl | - |
| CH₃ | CH₃ | 3-Cl-phenyl | - |
| CH₃ | CH₃ | 3-CH₃-phenyl | - |
| CH₃ | CH₃ | 3-NO₂-phenyl | - |
| CH₃ | CH₃ | 4-F-phenyl | - |
| CH₃ | CH₃ | 4-CH₃O-phenyl | - |
| CH₃ | CH₃ | 4-(*n*-Bu)-phenyl | - |
| | | | |
| -(CH₂)₄- | | phenyl | - |
| -(CH₂)₄- | | 2-CH₃-phenyl | - |
| -(CH₂)₅- | | 2-CH₃-phenyl | - |
| -(CH₂)₂O(CH₂)₂- | | phenyl | - |
| -(CH₂)₂O(CH₂)₂- | | 2-F-phenyl | - |
| -(CH₂)₂O(CH₂)₂- | | 2-CH₃-phenyl | - |
| -(CH₂)₂O(CH₂)₂- | | 2-CF₃-phenyl | - |
| -(CH₂)₂O(CH₂)₂- | | 2-CH₃CH₂-phenyl | - |
| -(CH₂)₂O(CH₂)₂- | | 2-CH₃O-phenyl | - |
| -(CH₂)₂O(CH₂)₂- | | 2-CH₃S-phenyl | - |
| -(CH₂)₂O(CH₂)₂- | | 3-CH₃-phenyl | - |
| -(CH₂)₂O(CH₂)₂- | | 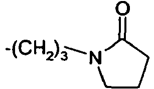 | - |
| -CH₂-CH(CH₃)OCH(CH₃)-(CH₃)-CH₂- | | 2-F-phenyl | - |
| -CH₂-CH(CH₃)OCH(CH₃)-CH₂- | | 2-CH₃O-phenyl | - |
| -CH₂-CH(CH₃)OCH(CH₃)-CH₂- | | 2-CH₃CH₂-phenyl | - |
| -(CH₂)₂S(CH₂)₂- | | 2-F-phenyl | - |
| -(CH₂)₂S(CH₂)₂- | | 2-CH₃CH₂-phenyl | - |
| -(CH₂)₂N(CH₃)(CH₂)₂- | | 2-CH₃-phenyl | - |
| | | | |
| CH₃ | - | Cl | CH₃ |
| CH₃ | - | CH₃ | CH₃ |
| CH₃ | - | 2-(*i*-Pr)-phenyl | CH₃ |
| CH₃ | - | 2-CH₃O-phenyl | CH₃ |
| CH₃ | - | 2,6-Cl₂-phenyl | CH₃ |
| CH₃ | - | 3-F-phenyl | CH₃ |
| CH₃ | - | 4-F-phenyl | CH₃ |
and tautomers, stereoisomers and N-oxides thereof, as well as pharmacologically acceptable salts, hydrates and solvates of said compounds of formula (I) and its tautomers, stereoisomers and N-oxides.

2. Compounds of the general formula (I): 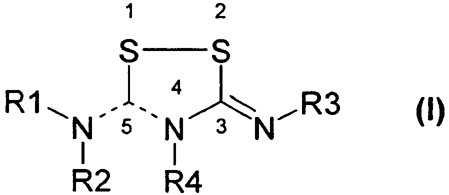 wherein
he dotted lines between carbon atom number 5 and its neighbouring nitrogen atoms represent single or double bonds, with the proviso that one of the two bonds is a single bond and one is a double bond, and the proviso that when the double bond is between carbon atom number 5 and nitrogen atom number 4, R4 does not exist and when the double bond is between carbon atom number 5 and the exo-nitrogen atom, R2 does not exist,
R1, R2, R3 and R4 independently represent a branched or unbranched alkyl(C₁₋₄) group, optionally containing sulphur atoms or sulphoxide, amide, ketone, thioketone or sulfone groups, and optionally substituted with halogen, cyano, mono- or dialkyl(C₁₋₄)amino, trifluoromethyl, (C₁₋₄)-alkoxy or hydroxyl, or
R1 and R2 together with the nitrogen atom to which they are attached, form a 4 to 8 membered ring system in which other (substituted) hetero atoms chosen from N, O and S may be present, and which ring system may be optionally substituted with branched or unbranched alkyl(C₁₋₄), halogen, cyano, mono- or dialkyl(C₁₋₄)amino, trifluoromethyl, (C₁₋₄)-alkoxy or hydroxyl or which ring system contains amide, ketone, thioketone, sulfone or sulfoxide functions,
R3 represents hydrogen, a branched or unbranched alkyl(C₁₋₄) group, optionally containing sulphur atoms or sulphoxide, amide, ketone, thioketone or sulfone groups, and optionally substituted with halogen, cyano, mono- or dialkyl(C₁₋₄)amino, trifluoromethyl, (C₁₋₄)alkoxy, S-alkyl(C₁₋₄), SH or hydroxyl, or
R3 represents an aryl group, optionally substituted with branched or unbranched alkyl(C₁₋₄), aryl, alkyl(C₁₋₄)aryl, SH, S-alkyl(C₁₋₄), halogen, cyano, mono- or dialkyl(C₁₋₄)amino, CF₃, OCF₃, SCF₃, nitro, hydroxy or (C₁₋₄)alkoxy groups, and tautomers, stereoisomers and N-oxides thereof, as well as pharmacologically acceptable salts, hydrates and solvates of said compounds of formula (I) and its tautomers, stereoisomers and N-oxides, for use in the preparation of a pharmaceutical composition for prophylaxis and treatment of neurodegenerative diseases and other diseases where free radical mediated cell degeneration and/or cell death (apoptosis) plays a decisive role.

3. A pharmaceutical composition comprising, in addition to a pharmaceutically acceptable carrier and/or at least one pharmaceutically acceptable auxiliary substance, a pharmacologically active amount of at least one compound as claimed in claim 1, or a salt thereof, as an active ingredient.

4. A method of preparing pharmaceutical compositions as claimed in claim 3, **characterized in that** a compound as claimed in claim 1 is brought into a form suitable for administration.

5. A compound as claimed in claim 1, or a salt thereof, for use as a medicament

6. Compound as claimed in claim 1 for use in the preparation of a pharmaceutical composition for prophylaxis and treatment of neurodegenerative diseases and other diseases where free radical mediated cell degeneration and/or cell death (apoptosis) plays a decisive role.

7. Use as claimed in claim 2 or claim 6, **characterized in that** said neurodegenerative diseases and other diseases where free radical mediated cell degeneration and/or cell death (apoptosis) plays a decisive role are neurodegenerative disorders such as ischemic stroke, traumatic brain injury, acute disseminated encephalomyelitis, amyotrophic lateral sclerosis (ALS), retinitis pigmentosa, mild cognitive impairment, Alzheimer's disease, Pick's disease, senile dementia, progressive supranuclear palsy, subcortical dementias, Wilson disease, multiple infarct disease, arteriosclerotic dementia, AIDS associated dementia, cerebellar degeneration, spinocerebellar degeneration syndromes, Friedreichs ataxia, ataxia telangiectasia, epilepsy related brain damage, spinal cord injury, restless legs syndrome, Huntington's disease and Parkinson's disease, striatonigral degeneration, cerebral vasculitis, mitochondrial encephalo-myopathies, neuronal ceroid lipofuscinosis, spinal muscular atrophies, lysosomal storage disorders with central nervous system involvement, leukodystrophies, urea cycle defect disorders, hepatic encephalopathies, renal encephalopathies, metabolic encephalopathies, porphyria, bacterial or viral meningitis and meningoencephalitis, prion diseases, poisonings with neurotoxic compounds, Guillain Barre syndrome, chronic inflammatory neuropathies, polymyositis, dermatomyositis, radiation-induced brain damage; irritable bowel disease and inflammatory bowel diseases, Crohn's disease and ulcerative colitis, coeliac disease, Helicobacter pylori gastritis and other infectious gastritides, necrotizing enterocolitis, pseudomembranous enterocolitis, radiation-induced enterocolitis, lymphocytic gastritis, graft-versus-host disease, acute and chronic pancreatitis; hepatic diseases such as alcoholic hepatitis, viral hepatitis, metabolic hepatitis, autoimmune hepatitis, radiation-induced hepatitis, liver cirrhosis, hemolytic uremic syndrome, glomerulonephritis, lupus nephritis, viral diseases such fulminant hepatitis: joint-diseases such as trauma and osteoarthritis; immuno-suppression or immunodeficiency, in particular autoimmune diseases like idiopathic inflammatory myopathy, chronic neutropenia, thrombotic thrombocytopenic purpura, rheumatoid arthritis, idiopathic thrombocytopenic purpura, autoimmune haemolytic syndromes, antiphospholipid antibody syndromes, myocarditis, multiple sclerosis and its diagnostic sub-classifications relapsing-remitting multiple sclerosis, secondary progressive multiple sclerosis, primary progressive multiple sclerosis, progressive relapsing multiple sclerosis, acute multiple sclerosis, benign relapsing multiple sclerosis or asymptomatic multiple sclerosis, neuromyelitis optica (Devic's syndrome), lymphocytic hypophysitis, Grave's disease, Addison's disease, hypoparathyroidism, type 1 diabetes, systemic lupus erythematodes, pemphigus vulgaris, bullous pemphigoid, psoriatic arthritis, endometriosis, autoimmune orchitis, autoimmune erectile dysfunction, sarcoidosis, Wegener's granulomatosis, autoimmune deafness, Sjögren's disease, autoimmune uveoretinitis, interstitial cystitis, Goodpasture's syndrome and fibromyalgia; myelodysplasias such as aplastic anemia; dermatological diseases including pemphigous vulgaris, dermatomyositis, atopic dermatitis, Henoch-Schonlein purpura, acne, systemic sclerosis, seborrhoeic keratosis, cutaneous mastocytosis, chronic proliferative dermatitis, dyskeratosis, scleroderma, interstitial granulomatous dermatitis, psoriasis, bacterial infections of the skin, dermatomycoses, lepra, cutaneous leishmaniasis, vitiligo, toxic epidermal necrolysis, Steven Johnson syndrome, sebaceous adenoma, alopecia, photodamage of the skin, lichen sclerosus, acute cutaneous wounds, incontinentia pigmenti, thermal damage of the skin, exanthematous pustulosis, lichenoid dermatosis, cutaneous allergic vasculitis, cytotoxic dermatitis; diseases of the inner ear such as acoustic trauma-induced auditory hair cell death and hearing loss, aminoglycoside induced auditory hair cell death and hearing loss, ototoxic drug-induced hearing loss, perilymphatic fistula, cholesteatoma, cochlear or vestibular ischemia, Meniere's disease, radiation-induced hearing loss, hearing loss induced by bacterial or viral infections and idiopathic hearing loss; transplantation: graft-versus-host disease, acute and chronic rejection of heart-, lung-, kidney-, skin-, corneal-, bone marrow- or liver-transplants; wound healing and tissue rejection.

## Patentansprüche

1. Verbindungen der allgemeinen Formel (I): 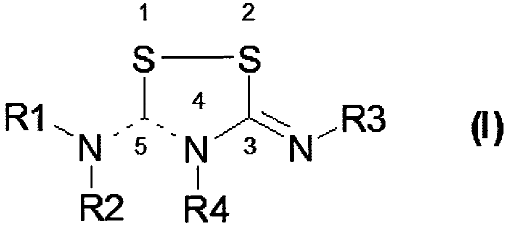 wobei die punktierten Linien für Einfach- oder Doppelbindungen stehen, ausgewählt aus der Gruppe bestehend aus Verbindungen mit den Substituenten:
| R1 | R2 | R3 | R4 |
|---|---|---|---|
| | | | |
| CH₃ | CH₃ | 2-Pyridyl | - |
| CH₃ | CH₃ | 3-Pyridyl | - |
| CH₃ | CH₃ | 2-F-Phenyl | - |
| CH₃ | CH₃ | 2-CH₃-Phenyl | - |
| CH₃ | CH₃ | 2-CF₃-Phenyl | - |
| CH₃ | CH₃ | 2-CH₃CH₂-Phenyl | - |
| CH₃ | CH₃ | 2- (*i*-Pr)-Phenyl | - |
| CH₃ | CH₃ | 2-CH₃O-Phenyl | - |
| CH₃ | CH₃ | 2-CH₃S-Phenyl | - |
| CH₃ | CH₃ | 2-Benzyl-Phenyl | - |
| CH₃ | CH₃ | 2, 6-Cl₂-Phenyl | - |
| CH₃ | CH₃ | 2, 6- (CH₃)₂-Phenyl | - |
| CH₃ | CH₃ | 3-Cl-Phenyl | - |
| CH₃ | CH₃ | 3-CH₃-Phenyl | - |
| CH₃ | CH₃ | 3-NO₂-Phenyl | - |
| CH₃ | CH₃ | 4-F-Phenyl | - |
| CH₃ | CH₃ | 4-CH₃O-Phenyl | - |
| CH₃ | CH₃ | 4-(*n*-Bu)-Phenyl | - |
| | | | |
| -(CH₂)₄- | | Phenyl | - |
| - (CH₂)₄- | | 2-CH₃-Phenyl | - |
| - (CH₂)₅- | | 2-CH₃-Phenyl | - |
| -(CH₂)₂O(CH₂)₂- | | Phenyl | - |
| -(CH₂)₂O(CH₂)₂- | | 2-F-Phenyl | - |
| -(CH₂)₂O(CH₂)₂- | | 2-CH₃-Phenyl | - |
| -(CH₂)₂O(CH₂)₂- | | 2-CF₃-Phenyl | - |
| -(CH₂)₂O(CH₂)₂- | | 2-CH₃CH₂-Phenyl | - |
| -(CH₂)₂O(CH₂)₂- | | 2-CH₃O-Phenyl | - |
| -(CH₂)₂O(CH₂)₂- | | 2-CH₃S-Phenyl | - |
| -(CH₂)₂O(CH₂)₂- | | 3-CH₃-Phenyl | - |
| -(CH₂)₂O(CH₂)₂- | | 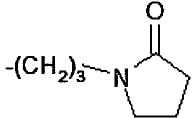 | - |
| -CH₂-CH (CH₃) OCH (CH₃) -CH₂- | | 2-F-Phenyl | - |
| -CH₂-CH (CH₃)OCH (CH₃) -CH₂- | | 2-CH₃O-Phenyl | - |
| -CH₂-CH(CH₃)OCH(CH₃)-CH₂- | | 2-CH₃CH₂-Phenyl | - |
| -(CH₂)₂S(CH₂)₂- | | 2-F-Phenyl | - |
| -(CH₂)₂S(CH₂)₂- | | 2-CH₃CH₂-Phenyl | - |
| -(CH₂)₂N(CH₃)(CH₂)₂- | | 2-CH₃-Phenyl | - |
| | | | |
| CH₃ | - | Cl | CH₃ |
| CH₃ | - | CH₃ | CH₃ |
| CH₃ | - | 2- (*i*-Pr) -Phenyl | CH₃ |
| CH₃ | - | 2-CH₃O-Phenyl | CH₃ |
| CH₃ | - | 2,6-Cl₂-Phenyl | CH₃ |
| CH₃ | - | 3-F-Phenyl | CH₃ |
| CH₃ | - | 4-F-Phenyl | CH₃ |
und Tautomere, Stereoisomere und N-Oxide davon, sowie pharmakologisch akzeptable Salze, Hydrate und Solvate der Verbindungen der Formel (I) und deren Tautomere, Stereoisomere und N-Oxide.

2. Verbindungen der allgemeinen Formel (I): 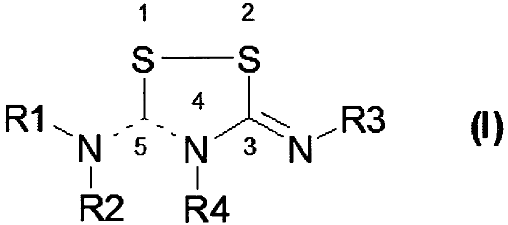 wobei
die punktierten Linien zwischen Kohlenstoffatom Nummer 5 und dessen benachbarten Stickstoffatomen für Einfach- oder Doppelbindungen stehen, unter der Voraussetzung, dass eine der zwei Bindungen eine Einfachbindung ist und eine eine Doppelbindung ist und unter der Voraussetzung, dass, wenn die Doppelbindung zwischen Kohlenstoffatom Nummer 5 und Stickstoffatom Nummer 4 ist, R4 nicht vorhanden ist und wenn die Doppelbindung zwischen Kohlenstoffatom Nummer 5 und dem exo-Stickstoffatom ist, R2 nicht vorhanden ist,
R1, R2, R3 und R4 unabhängig voneinander für eine verzweigte oder nicht-verzweigte Alkyl(C₁₋₄)-Gruppe stehen, die gegebenenfalls Schwefelatome oder Sulfoxid-, Amid-, Keton-, Thioketon- oder Sulfongruppen enthält und gegebenenfalls mit Halogen, Cyano, Mono- oder Dialkyl(C₁₋₄) -Amino, Trifluormethyl, (C₁₋₄)-Alkoxy oder Hydroxyl substituiert ist, oder
R1 und R2 gemeinsam mit dem Stickstoffatom, an das sie gebunden sind, ein 4- bis 8-gliedriges Ringsystem bilden, in dem andere (substituierte) Heteroatome ausgewählt aus N, O und S vorhanden sein können, und wobei das Ringsystem gegebenenfalls mit verzweigtem oder nicht-verzweigtem Alkyl(C₁₋₄), Halogen, Cyano, Mono- oder Dialkyl(C₁₋₄)-Amino, Trifluormethyl, (C₁₋₄)-Alkoxy oder Hydroxyl substituiert sein kann oder wobei das Ringsystem Amid-, Keton-, Thioketon-, Sulfon- oder Sulfoxidfunktionen enthält,
R3 für Wasserstoff, eine verzweigte oder nicht-verzweigte Alkyl(C₁₋₄)-Gruppe steht, die gegebenenfalls Schwefelatome oder Sulfoxid-, Amid-, Keton-, Thioketon- oder Sulfongruppen enthält und gegebenenfalls mit Halogen, Cyano, Mono- oder Dialkyl(C₁₋₄)-Amino, Trifluormethyl, (C₁₋₄)-Alkoxy, S-Alkyl(C₁₋₄), SH oder Hydroxyl substituiert ist, oder
R3 für eine Arylgruppe steht, die gegebenenfalls mit verzweigten oder nicht-verzweigten Alkyl(C₁₋₄)-, Aryl-, Alkyl(C₁₋₄)-Aryl-, SH-, S-Alkyl(C₁₋₄)-, Halogen-, Cyano-, Mono- oder Dialkyl(C₁₋₄)-Amino-, CF₃-, OCF₃-, SCF₃-, Nitro-, Hydroxy- oder (C₁₋₄)-Alkoxygruppen substituiert ist und Tautomere, Stereoisomere und N-Oxide davon, sowie pharmakologisch akzeptable Salze, Hydrate und Solvate der Verbindungen der Formel (I) und deren Tautomere, Stereoisomere und N-Oxide, für die Verwendung bei der Herstellung einer pharmazeutischen Zusammensetzung zur Prophylaxe und Behandlung von neurodegenerativen Erkrankungen und anderen Erkrankungen bei denen durch freie Radikale vermittelte Zelldegeneration und/oder Zelltod (Apoptose) eine entscheidende Rolle spielt.

3. Pharmazeutische Zusammensetzung, die zusätzlich zu einem pharmazeutisch akzeptablen Träger und/oder zumindest einer pharmazeutisch akzeptablen Hilfssubstanz, eine pharmakologisch aktive Menge von zumindest einer Verbindung wie in Anspruch 1 beansprucht, oder ein Salz davon, als Wirkstoff enthält.

4. Verfahren zur Herstellung pharmazeutischer Zusammensetzungen wie in Anspruch 3 beansprucht, **dadurch gekennzeichnet, dass** eine wie in Anspruch 1 beanspruchte Verbindung in eine für die Verabreichung geeignete Form gebracht wird.

5. Verbindung, wie in Anspruch 1 beansprucht, oder ein Salz davon, zur Verwendung als Medikament.

6. Verbindung, wie in Anspruch 1 beansprucht, für die Verwendung bei der Herstellung einer pharmazeutischen Zusammensetzung zur Prophylaxe und Behandlung von neurodegenerativen Erkrankungen und anderen Erkrankungen, bei denen durch freie Radikale vermittelte Zelldegeneration und/oder Zelltod (Apoptose) eine entscheidende Rolle spielt.

7. Verwendung, wie in Anspruch 2 oder Anspruch 6 beansprucht, **dadurch gekennzeichnet, dass** die neurodegenerativen Erkrankungen und anderen Erkrankungen, bei denen durch freie Radikale vermittelte Zelldegeneration und/oder Zelltod (Apoptose) eine entscheidende Rolle spielt, neurodegenerative Erkrankungen sind, wie zum Beispiel ischämischer Schlaganfall, Schädel-Hirn-Trauma, akute disseminierte Enzephalomyelitis, amyotrophe Lateralsklerose (ALS), Retinitis pigmentosa, milde kognitive Beeinträchtigung, Alzheimer-Krankheit, Pick-Krankheit, senile Demenz, progressive supranukleäre Blickparese, subkortikale Demenzen, Wilson-Krankheit, Multi-Infarkt-Erkrankung, arteriosklerotische Demenz, AIDS-assoziierte Demenz, zerebelläre Degeneration, spinozerebelläre Ataxien, Friedreich-Ataxie, Ataxie-Telangiektasie, mit Epilepsie in Zusammenhang stehender Gehirnschaden, Rückenmarksverletzung, Restless-Legs-Syndrom, Huntington-Krankheit und Parkinson-Krankheit, striatonigrale Degeneration, zerebrale Vaskulitis, mitochondriale Enzephalomyopathien, neuronale Zeroidlipofuszinose, spinale Muskelatrophien, Lysosomale Speicherkrankheit mit Beteiligung des zentralen Nervensystems, Leukodystrophien, Harnstoffzyklusstörungen, hepatische Enzephalopathien, renale Enzephalopathien, metabolische Enzephalopathien, Porphyrie, bakterielle oder virale Meningitis und Meningoenzephalitis, Prionerkrankung, Vergiftungen mit neurotoxischen Verbindungen, Guillain-Barré-Syndrom, chronisch inflammatorische Neuropathien, Polymyositis, Dermatomyositis, strahleninduzierter Gehirnschaden; Reizdarm und entzündliche Darmerkrankungen, Morbus Crohn und Colitis ulcerosa, Zöliakie, Helicobacter-pylori-Gastritis und andere infektiöse Gastritiden, nekrotisierende Enterokolitis, pseudomembranöse Enterokolitis, strahleninduzierte Enterokolitis, lymphozytäre Gastritis, Graft-versus-host-Krankheit, akute und chronische Pankreatitis; hepatische Erkrankungen, wie zum Beispiel alkoholische Hepatitis, virale Hepatitis, metabolische Hepatitis, autoimmune Hepatitis, strahleninduzierte Hepatitis, Leberzirrhose, hämolytisch-urämisches Syndrom, Glomerulonephritis, Lupus Nephritis, Virenerkrankungen, wie zum Beispiel fulminante Hepatitis: Gelenkerkrankungen, wie zum Beispiel Trauma und Osteoarthritis; Immunsuppression oder Immundefizienz, insbesondere autoimmune Erkrankungen, wie z.B. idiopathische inflammatorische Myopathie, chronische Neutropenie, thrombotisch-thromozytopenische Purpura, rheumatoide Arthritis, idiopathische thrombozytopenische Purpura, autoimmune hämolytische Syndrome, Antiphospholipid-Antikörper-Syndrome, Myokarditis, multiple Sklerose und ihre diagnostischen Untergruppen: schubförmig remittierende multiple Sklerose, sekundär progrediente multiple Sklerose, primär progrediente multiple Sklerose, progrediente schubförmige multiple Sklerose, akute multiple Sklerose, benigne schubförmige multiple Sklerose oder asymptomatische multiple Sklerose, Neuromyelitis optica (Devic-Syndrom), lymphozytäre Hypophysitis, Morbus Basedow, Addison-Krankheit, Hyperparathyreoidismus, Typ-1-Diabetes, systemischer Lupus erythematodes, Pemphigus vulgaris, bullöses Pemphigoid, psoriatische Arthritis, Endometriose, autoimmune Orchitis, autoimmune erektile Dysfunktion, Sarkoidose, Wegener-Granulomatose, autoimmune Gehörlosigkeit, Sjögren-Syndrom, autoimmune Uveoretinitis, interstitielle Cystitis, Goodpasture-Syndrom und Fibromyalgie; Myelodysplasien, wie zum Beispiel aplastische Anämie; dermatologische Erkrankungen einschließlich Pemphigus vulgaris, Dermatomyositis, atopische Dermatitis, Purpura Schönlein-Henoch, Akne, systemische Sklerose, seborrhoische Keratose, kutane Mastozytose, chronisch proliferative Dermatitis, Dyskeratose, Sklerodermie, interstitielle granulomatöse Dermatitis, Psoriasis, bakterielle Infektionen der Haut, Dermatomykose, Lepra, kutane Leishmaniasis, Weißfleckenkrankheit, toxische epidermale Nekrolyse, Stevens-Johnson-Syndrom, Talgdrüsenadenom, Alopezie, Lichtschäden an der Haut, Lichen sclerosus, akute Hautwunden, Bloch-Sulzberger-Syndrom, thermische Hautschäden, exanthematische Pustulose, lichenoide Dermatose, allergische Hautvaskulitis, zytotoxische Dermatitis; Erkrankungen des Innenohrs, wie zum Beispiel akustischer traumainduzierter Haarzelltod und Gehörverlust, aminoglykosidinduzierter Haarzelltod und Gehörverlust, ototoxischer arzneimittelinduzierter Gehörverlust, Perilymphfistel, Cholesteatom, cochleäre oder vestibuläre Ischämie, Morbus Menière, strahleninduzierter Gehörverlust, Gehörverlust induziert durch bakterielle oder virale Infektionen und idiopathischer Gehörverlust; Transplantation: Graft-versus-host-Krankheit, akute und chronische Abstoßung von Herz-, Lungen-, Nieren-, Haut-, Hornhaut-, Knochenmark- oder Lebertransplantaten; Wundheilung und Gewebeabstoßung.

## Revendications

1. Composés de formule générale (I) : 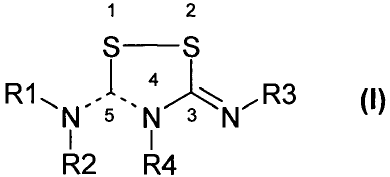 dans laquelle les lignes de tirets représentent des liaisons simples ou doubles, choisis dans le groupe constitué par les composés ayant les substituants suivants :
| **R1** | **R2** | **R3** | **R4** |
|---|---|---|---|
| | | | |
| CH₃ | CH₃ | 2-pyridyle | - |
| CH₃ | CH₃ | 3-pyridyle | - |
| CH₃ | CH₃ | 2-F-phényle | - |
| CH₃ | CH₃ | 2-CH₃-phényle | - |
| CH₃ | CH₃ | 2-CF₃-phényle | - |
| CH₃ | CH₃ | 2-CH₃CH₂-phényle | - |
| CH₃ | CH₃ | 2-(*i*-Pr)-phényle | - |
| CH₃ | CH₃ | 2-CH₃O-phényle | - |
| CH₃ | CH₃ | 2-CH₃S-phényle | - |
| CH₃ | CH₃ | 2-benzyle-phényle | - |
| CH₃ | CH₃ | 2,6-Cl₂-phényle | - |
| CH₃ | CH₃ | 2,6-(CH₃)₂-phényle | - |
| CH₃ | CH₃ | 3-Cl-phényle | - |
| CH₃ | CH₃ | 3-CH₃-phényle | - |
| CH₃ | CH₃ | 3-NO₂-_{P}hényle | - |
| CH₃ | CH₃ | 4-F-phényle | - |
| CH₃ | CH₃ | 4-CH₃O-phényle | - |
| CH₃ | CH₃ | 4-(*n*-Bu)-phényle | - |
| | | | |
| -(CH₂)₄- | | phényle | - |
| -(CH₂)₄- | | 2-CH₃-phényle | - |
| -(CH₂)₅- | | 2-CH₃-phényle | - |
| -(CH₂)₂O(CH₂)₂- | | phényle | - |
| -(CH₂)₂O(CH₂)₂- | | 2-F-phényle | - |
| -(CH₂)₂O(CH₂)₂- | | 2-CH₃-phényle | - |
| -(CH₂)₂O(CH₂)₂- | | 2-CF₃-phényle | - |
| -(CH₂)₂O(CH₂)₂- | | 2-CH₃CH₂-phényle | - |
| -(CH₂)₂O(CH₂)₂- | | 2-CH₃O-phényle | - |
| -(CH₂)₂O(CH₂)₂- | | 2-CH₃S-phényle | - |
| -(CH₂)₂O(CH₂)₂- | | 3-CH₃-phényle | - |
| -(CH₂)₂O(CH₂)₂- | | 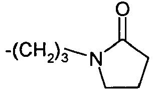 | - |
| -CH₂-CH(CH₃)OCH(CH₃)-CH₂- | | 2-F-phényle | - |
| -CH₂-CH(CH₃)OCH(CH₃)-CH₂- | | 2-CH₃O-phényle | - |
| -CH₂-CH(CH₃)OCH(CH₃)-CH₂- | | 2-CH₃CH₂-phényle | - |
| -(CH₂)₂S(CH₂)₂- | | 2-F-phényle | - |
| -(CH₂)₂S(CH₂)₂- | | 2-CH₃CH₂-phényle | - |
| -(CH₂)₂N(CH₃)(CH₂)₂- | | 2-CH₃-phényle | - |
| | | | |
| CH₃ | - | Cl | CH₃ |
| CH₃ | - | CH₃ | CH₃ |
| CH₃ | - | 2-(*i*-Pr)-phényle | CH₃ |
| CH₃ | - | 2-CH₃O-phényle | CH₃ |
| CH₃ | - | 2,6-Cl₂-phényle | CH₃ |
| CH₃ | - | 3-F-phényle | CH₃ |
| CH₃ | - | 4-F-phényle | CH₃ |
et leurs tautomères, stéréoisomères et N-oxydes, ainsi que les sels pharmacologiquement acceptables, hydrates et solvates desdits composés de formule (I) et leurs tautomères, stéréoisomères et N-oxydes.

2. Composés de formule générale (I) : 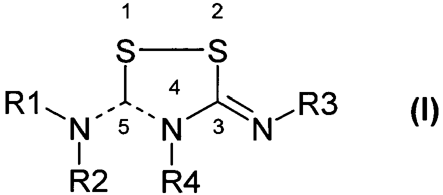 dans laquelle les lignes de tirets entre l'atome de carbone N° 5 et ses atomes d'azote voisins représentent des liaisons simples ou doubles, sous réserve que l'une des deux liaisons soit une liaison simple et l'autre soit une double liaison, et sous réserve que, lorsque la double liaison est entre l'atome de carbone N° 5 et l'atome d'azote N° 4, R4 n'existe pas et que, lorsque la double liaison est entre l'atome de carbone N° 5 et l'exo-atome d'azote, R2 n'existe pas,
R1, R2, R3 et R4 représentent indépendamment un groupe alkyle en C₁ à C₄ ramifié ou non ramifié, contenant éventuellement des atomes de soufre ou des groupes sulfoxyde, amide, cétone, thiocétone ou sulfone, et éventuellement substitué par un halogène ou un radical cyano, mono- ou di-alkylamino en C₁ à C₄, trifluorométhyle, alcoxy en C₁ à C₄ ou hydroxyle, ou bien
R1 et R2, ensemble avec l'atome d'azote auquel ils sont rattachés, forment un système cyclique à 4 à 8 chaînons dans lequel d'autres hétéroatomes (substitués) choisis parmi N, O et S peuvent être présents, lequel système cyclique pouvant être éventuellement substitué par un halogène ou un radical alkyle en C₁ à C₄ ramifié ou non ramifié, cyano, mono- ou di-alkylamino en C₁ à C₄, trifluorométhyle, alcoxy en C₁ à C₄ ou hydroxyle, ou bien lequel système cyclique contenant des fonctionnalités amide, cétone, thiocétone, sulfone ou sulfoxyde,
R3 représente l'hydrogène, un halogène ou un groupe alkyle en C₁ à C₄ ramifié ou non ramifié, contenant éventuellement des atomes de soufre ou des groupes sulfoxyde, amide, cétone, thiocétone ou sulfone, et éventuellement substitué par un halogène, groupe cyano, mono- ou di-alkylamino en C₁ à C₄, trifluorométhyle, alcoxy en C₁ à C₄, S-alkyl en C₁ à C₄, SH ou hydroxyle, ou bien
R3 représente un groupe aryle, éventuellement substitué par un halogène ou un groupe alkyle en C1 à C4 ramifié ou non ramifié, aryle, alkylaryle en C₁ à C₄, SH, -S-alkyle en C₁ à C₄, cyano, mono- ou di-alkylamino en C₁ à C₄, CF₃, OCF₃, SCF₃, nitro, hydroxy ou alcoxy en C₁ à C₄, et leurs tautomères, stéréoisomères et N-oxydes, ainsi que les sels pharmacologiquement acceptables, hydrates et solvates desdits composés de formule (I) et leurs tautomères, stéréoisomères et N-oxydes, pour utilisation dans la préparation d'une composition pharmaceutique destinée à la prophylaxie ou au traitement de maladies neurodégénératives et d'autres maladies où une mort cellulaire (apoptose) et/ou une dégénérescence cellulaire à médiation par des radicaux libres jouent un rôle décisif.

3. Composition pharmaceutique comprenant, en plus d'un véhicule pharmaceutiquement acceptable et/ou d'au moins une substance auxiliaire pharmaceutiquement acceptable, une quantité active, du point de vue pharmacologique, d'au moins un composé selon la revendication 1, ou d'un sel de celui-ci, à titre d'ingrédient actif.

4. Procédé pour préparer des compositions pharmaceutiques selon la revendication 3, **caractérisé en ce qu'**un composé selon la revendication 1 est mis sous une forme convenant pour une administration.

5. Composé selon la revendication 1, ou sel de celui-ci, pour utilisation en tant que médicament.

6. Composé selon la revendication 1 pour utilisation dans la préparation d'une composition pharmaceutique destinée à la prophylaxie ou au traitement de maladies neurodégénératives et d'autres maladies où une mort cellulaire (apoptose) et/ou une dégénérescence cellulaire à médiation par des radicaux libres jouent un rôle décisif.

7. Utilisation selon la revendication 2 ou la revendication 6, **caractérisée en ce que** lesdites maladies neurodégénératives et autres maladies où une mort cellulaire (apoptose) et/ou une dégénérescence cellulaire à médiation par des radicaux libres jouent un rôle décisif sont des troubles neurodégénératifs tels qu'un accident ischémique, une lésion cérébrale traumatique, une encéphalomyélite aiguë post-infectieuse, une sclérose latérale amyotrophique (SLA), une rétinite pigmentaire, une altération cognitive légère, la maladie d'Alzheimer, la maladie de Pick, une démence sénile, une ophtalmoplégie supranucléaire progressive, une démence sous-corticale, la maladie de Wilson, un infarctus multiple, une démence artériosclérotique, une démence associée au SIDA, une dégénérescence cérébelleuse, un syndrome de dégénérescence spinocérébelleuse, l'ataxie de Friedreich, une ataxie télangiectasie, un dommage cérébral lié à l'épilepsie, une lésion de la moelle épinière, le syndrome des jambes sans repos, la maladie de Huntington et la maladie de Parkinson, une dégénérescence striatonigrique, une angéite cérébrale, une encéphalomyopathie mitochondriale, une lipofuscinose céroïde neuronale, une amyotrophie spinale, des troubles de stockage des lysosomes impliquant le système nerveux central, une leucodystrophie, des défauts du cycle de l'urée, une encéphalopathie hépatique, une encéphalopathie rénale, une encéphalopathie métabolique, une porphyrie, une méningite ou méningo-encéphalite bactérienne ou virale, les maladies liées aux prions, un empoisonnement avec des composés neurotoxiques, le syndrome de Guillain Barré, une neuropathie inflammatoire chronique, une polymyosite, une dermatomyosite, un dommage cérébral induit par des radiations ; le syndrome du côlon irritable et une maladie intestinale inflammatoire, la maladie de Crohn et la recto-colite hémorragique, une maladie coeliaque, une gastrite à Helicobacter pylori et autres gastritides infectieux, une entérocolite nécrosante, une entérocolite pseudomembraneuse, une entérocolite induite par des radiations, une gastrite lymphocytaire, une réaction du greffon contre l'hôte, une pancréatite aiguë ou chronique ; une maladie hépatique telle qu'une hépatite alcoolique, une hépatite virale, une hépatite métabolique, une hépatite autoimmune, une hépatite induite par des radiations, une cirrhose du foie, le syndrome urémique hémolytique, une glomérulonéphrite, un lupus néphritique, une maladie virale telle qu'une hépatite fulminante ; une maladie articulaire telle qu'un trauma ou l'arthrose ; une immunodépression ou une immunodéficience, en particulier une maladie auto-immune telle qu'une myopathie inflammatoire idiopathique, une neutropénie chronique, un purpura thrombocytopénique thrombotique, la polyarthrite rhumatoïde, un purpura thrombocytopénique idiopathique, un syndrome hémolytique auto-immun, un syndrome d'anticorps antiphospholipides, une myocardite, la sclérose en plaques et ses sous-classifications de diagnostic de sclérose en plaques cyclique, sclérose en plaques progressive secondaire, sclérose en plaques progressive primaire, sclérose en plaques progressive rémittente, sclérose en plaques aiguë, sclérose en plaques rémittente bénigne ou sclérose en plaques asymptomatique, une neuromyélite optique (syndrome de Devic), une hypophysite lymphocytaires, la maladie de Grave, la maladie d'Addison, une hypoparathyroïdie, le diabète de type 1, un lupus érythémateux disséminé, un pemphigus vulgaire, une pemphigoïde bulleuse, l'arthrite psoriasique, une endométriose, une orchite auto-immune, un dysfonctionnement érectile auto-immun, une sarcoïdose, la granulomatose de Wegener, une surdité auto-immune, la maladie de Sjôgren, une uvéorétinite autoimmune, une cystite interstitielle, le syndrome de Goodpasture et une fibromyalgie ; une myélodysplasie telle qu'une anémie aplasique ; une maladie dermatologique, y compris un pemphigus vulgaire, une dermatomyosite, une dermatite atopique, un purpura de Henoch-Schonlein, l'acné, une sclérodermie généralisée, une kératose séborrhéique, une mastocytose cutanée, une dermatite proliférative chronique, une dyskératose, une sclérodermie, une dermatite granulomateuse interstitielle, un psoriasis, une infection bactérienne de la peau, une dermatomycose, la lèpre, une leishmaniose cutanée, un vitiligo, une nécrolyse épidermique toxique, le syndrome de Steven Johnson, un adénome sébacé, une alopécie, un dommage cutané dû à la lumière, un sclérolichen, une plaie cutanée aiguë, une incontinentia pigmenti, un dommage thermique de la peau, une pustulose exanthémateuse, une dermatose lichénoïde, une angéite allergique cutanée, une dermatite cytotoxique ; une maladie de l'oreille interne telle qu'une perte d'audition et une mort de cellules ciliées induites par un traumatisme acoustique, une perte d'audition et une mort de cellules ciliées induites par un aminoglycoside, une perte d'audition induite par un médicament ototoxique, une fistule périlymphatique, un cholestéatome, une ischémie cochléaire ou vestibulaire, la maladie de Meniere, une perte d'audition induite par une radiation, une perte d'audition induite par des infections bactériennes ou virales et une perte d'audition idiopathique ; une transplantation ; une réaction du greffon contre l'hôte, un rejet aigu ou chronique de greffe de coeur, poumon, rein, peau, cornée, moelle osseuse ou foie ; un rejet de tissu et de tissu cicatriciel.
